Europäisches Patentamt

European Patent Office

Office européen des brevets

(19)

(11) **EP 0 940 157 A2**

(12) **EUROPÄISCHE PATENTANMELDUNG**

(43) Veröffentlichungstag:
08.09.1999 Patentblatt 1999/36

(51) Int. Cl.⁶: **A61N 2/02**

(21) Anmeldenummer: 99100642.0

(22) Anmeldetag: 14.01.1999

(84) Benannte Vertragsstaaten:
AT BE CH CY DE DK ES FI FR GB GR IE IT LI LU
MC NL PT SE
Benannte Erstreckungsstaaten:
AL LT LV MK RO SI

(30) Priorität: 29.01.1998 DE 19803419
20.08.1998 DE 29814987 U

(71) Anmelder:
• Lorenzen, Hans Werner Prof.
81247 München (DE)
• WEYH, Thomas
W-8000 München 40 (DE)

• Vachenauer, Renate Dipl. Ing.
80799 München (DE)

(72) Erfinder:
• Lorenzen, Hans Werner Prof.
81247 München (DE)
• WEYH, Thomas
W-8000 München 40 (DE)
• Vachenauer, Renate Dipl. Ing.
80799 München (DE)

(74) Vertreter:
Viering, Jentschura & Partner
Postfach 22 14 43
80504 München (DE)

(54) **Vorrichtung zur Stimulation von Körpergewebe**

(57) Vorrichtung zur Stimulation von Körpergewebe mit einer auf eine Körperoberfläche aufsetzbaren Magnetspulenanordnung (12) und einer zur Erzeugung von Stromimpulsen in der Magnetspulenanordnung betreibbaren Erregereinrichtung (10, 14, 16), wobei die Magnetspulenanordnung (12) mindestens einen aus einer Mehrzahl von spiralenartig gewickelten Windungen bestehenden Spulenbereich aufweist.

Fig. 1

EP 0 940 157 A2

**Beschreibung**

Die Erfindung betrifft eine Vorrichtung zur Stimulation von Körpergewebe mit einer auf eine Körperoberfläche aufsetzbaren Magnetspulenanordnung und einer zur Erzeugung von Stromimpulsen in der Magnetspulenanordnung betreibbaren Erregereinrichtung.

Derartige Vorrichtungen werden beispielsweise zur induktiven Erregung von menschlichem Nerven- oder Muskelgewebe benutzt. Dabei wird mit der Erregereinrichtung ein Stromimpuls erzeugt, der durch die in der Nähe des zu stimulierenden Gewebes angeordnete Magnetspulenanordnung fließt. Dadurch wird in dem zu stimulierenden Gewebe ein sich zeitlich schnell veränderndes Magnetfeld erzeugt, das wiederum von einem sich ebenfalls zeitlich schnell verändernden elektrischen Feld begleitet wird. Durch das so im Gewebe induzierte elektrische Feld wird in den zu stimulierenden Gewebezellen ein Aktionspotential ausgelöst und so die gewünschte Stimulation bzw. der gewünschte Reizeffekt erreicht.

Bei einer aus der DE 39 04 254 A 1 bekannten Vorrichtung der eingangs beschriebenen Art, die zur Defibrillation des Herzens gedacht ist, wird eine im wesentlichen aus einem Kondensator großer Kapazität bestehende Erregereinrichtung verwendet. Die zur Stimulation benötigten Stromimpulse werden dabei dadurch erzeugt, daß der Kondensator über eine auf einen ferromagnetischen Kern gewickelte Magnetspule entladen wird. Bei der bekannten Vorrichtung bilden der Kondensator und die Magnetspulenanordnung einen Schwingkreis, in dem die genannten Elemente und die Leitungen Widerstände darstellen, in denen die elektrische Energie in Wärme umgewandelt wird. Dadurch wird zum einen die zur induktiven Erregung benötigte elektrische Energie erhöht und zum anderen eine unerwünschte Spulenerwärmung bei gegebenem Reizeffekt erzeugt. Insbesondere die starke Erwärmung der Spule führt bei der in letzter Zeit immer mehr an Bedeutung gewinnenden repetitiven Nerven- bzw. Muskelstimulation (d.h. Nervenreizung mit einer Wiederholungsrate von ca. 15 bis 40 Impulsen pro Sekunde) zu besonderen Problemen, weil die rasche Spulenerwärmung längere Reizserien kaum zuläßt. Aber auch der große Energieaufwand führt bei dieser Stimulationsmethode zu nicht unerheblichen Problemen, weil die Geräte dadurch sehr groß und teuer werden.

Ein wichtiges Einsatzgebiet der gerade beschriebenen Stimulationsmethode stellt die sogenannte Muskelstimulation dar (bei der tatsächlich nur die motorischen Nerven in unmittelbarer Nähe der jeweiligen Muskeln gereizt werden). Dabei kann durch die repetitive Stimulation eine Dauerkontraktion der jeweiligen Muskeln erreicht werden. Ein anderes interessantes Anwendungsgebiet der repetitiven Stimulation stellt die Stimulation der motorischen Großhirnrinde (Motor Cortex) und anderer Gehirnareale dar. Eine für dieses Einsatzgebiet konzipierte Vorrichtung der eingangs beschriebenen Art ist in J. Clin. Neurophysiol., Vol.12, Nr. 5, 1995 beschrieben. Zur Vermeidung einer unerwünschten Erwärmung der Magnetspulenanordnung wird in dieser Schrift die Verwendung einer 16 Windungen aufweisenden Spule in Form eines von einem Kühlmittel durchflossenen Kupferrohres mit einer Rohrwandungsquerschnittfläche von 14,8 mm$^2$ vorgeschlagen.

Mit dieser Magnetspulenanorndung ist eine beachtliche Reduzierung der Spulenerwärmung erreichbar. Dieser Vorteil wird mit der bekannten Vorrichtung jedoch nur erzielt, indem ein erhöhter Energieaufwand für die Kühlung der Magnetspulenanordnung und ein erhöhter apparativer Aufwand für die Bereitstellung eines entsprechenden Kühlmittelkreislaufs in Kauf genommen wird.

Angesichts der beschriebenen Probleme im Stand der Technik liegt dieser Erfindung die Aufgabe zugrunde, eine Vorrichtung der eingangs genannten angegebenen Art bereitzustellen, mit der ohne zusätzlichen apparativen und energetischen Aufwand bei gegebenem Reizeffekt eine unerwünschte Spulenerwärmung vermieden werden kann.

Erfindungsgemäß wird diese Aufgabe durch Verwendung einer Magnetspulenanordnung gelöst, die mindestens einen aus einer Mehrzahl von spiralenartig gewickelten Windungen bestehenden Spulenbereich aufweist.

Diese Lösung geht auf die Erkenntnis zurück, daß unter den den Energieverbrauch der bekannten Vorrichtungen beeinflußenden Faktoren, nämlich dem Stromwärmeverlust im Kondensator, dem Stromwärmeverlust in der Spule, den Verlusten in der Erregereinrichtung, der Energieabsorption im Körpergewebe und den Zuleitungsverlusten, bei gegebenem Reizeffekt lediglich die Stromwärmeverluste in der Spule eine weitere Optimierung als aussichtsreich erscheinen lassen, weil die übrigen Faktoren ohnehin nur einen geringen Einfluß auf den gesamten Energieverbrauch haben oder durch Auswahl entsprechend hochwertiger Kondensatoren bereits optimiert wurden. Weiter wurde erkannt, daß die Spulenanordnung bei vorgegebenem Reizeffekt optimiert werden kann, wenn anstelle der bislang üblichen, wendelförmig gewickelten Spulen eine spiralenartig gewickelte, also flache Spule eingesetzt wird, weil dadurch der Abstand zwischen den einzelnen stromdurchflossenen Windungen und dem zu Stimulierenden Gewebe reduziert werden kann, was letztlich zu einer Reduzierung des zur Erzeugung einer vorgegebenen Reizwirkung benötigten Spulenstroms und damit zu einer Reduzierung der Stromwärmeverluste in der Spule führt. Beim Einsatz einer erfindungsgemäßen Magnetspulenanordnung hat die im stimulierten Körpergewebe induzierte Stromdichteverteilung etwa die gleiche räumliche Verteilung wie die Stromdichte in der Spule.

Zur Erhöhung der Stimulationswirkung kann die erfindungsgemäße Magnetspulenanordnung auch zwei oder mehr Spulenbereiche aufweisen, von denen jeder aus einer Mehrzahl von spiralenartig gewickelten Windungen besteht. Diese Spulenbereiche können bezüglich einer gemeinsamen Spulenachse axial versetzt übereinander ange-

2

ordnet werden.

Als besonders zweckmäßig hat es sich jedoch erwiesen, wenn die Spulenbereiche bezüglich einer Spulenachse radial versetzt nebeneinander angeordnet sind und die Erregereinrichtung zur Erzeugung gleichsinniger Stromimpulse in einander unmittelbar benachbarten Segmenten der nebeneinander angeordneten Spulenbereiche betreibbar ist. Mit einer derartigen Doppelspule wird unterhalb der unmittelbar benachbarten Segmente das größte Feld und damit die stärkste Stimulationswirkung erzeugt, da sich in diesem Bereich eine große Anzahl gleichsinnig durchflossener Leiter direkt nebeneinander befinden. Eine derartige Doppelspule hat daher eine stärkere Stimulationswirkung als eine Rundspule gleicher Größenordnung. Ein weiterer Vorteil beim Einsatz der beschriebenen Doppelspule ist darin zu sehen, daß im Hinblick auf die Ähnlichkeit zwischen der Form des im Körpergewebe induzierten Stroms und der Form der Leiteranordnung in der Spule unterhalb der Außenbereiche der Doppelspule eine Rückführung des induzierten Gewebestroms mit gegenüber dem Zentrum der Spule reduzierter Stromdichte stattfindet.

Bei der Herstellung der beschriebenen Doppelspule hat es sich als besonders zweckmäßig erwiesen, wenn die nebeneinander angeordneten Spulenbereiche gleichsinnig gewickelt sind und die äußeren Windungen dieser Spulenbereiche vorzugsweise tangential ineinander übergehen.

Wie vorstehend bereits erläutert, läßt sich ein besonders hoher Wirkungsgrad der erfindungsgemäßen Vorrichtung erreichen, wenn im Bereich des zu stimulierenden Gewebes eine besonders hohe Stromdichte erzeugt wird, wahrend die Stromrückführung mit einer im Vergleich dazu geringeren Stromdichte erfolgt.

Anhand von Feldrechnungen und zahlreichen medizinischen Tests konnten die für die optimale Stimulationswirkung tatsachlich relevanten physikalischen Großen ermittelt und optimiert werden. Dabei hat sich herausgestellt, daß bei gegebenem Reizeffekt ein besonders hoher Wirkungsgrad erreichbar ist, wenn mindestens einer der aus spiralenartig gewickelten Windungen bestehenden Spulenbereiche mindestens zwei Winkelsegmente mit sich voneinander unterscheidender, integraler Stromdichte aufweist. Dabei bezeichnet die integrale Stromdichte den Quotienten aus dem Produkt aus Spulenstrom und Windungszahl einerseits und der in dem entsprechenden Winkelsegment von den Windungen durchsetzten Flache. Auch bei dieser Anordnung wird davon Gebrauch gemacht, daß am Ort der Stimulation unterhalb des Segmentes mit einer höheren integralen Stromdichte eine Erhöhung des Feldgradienten und der Feldenergiedichte erreicht wird während beide Großen gleichzeitig in den Außenbereichen gesenkt werden. Durch die Senkung des Feldgradienten und der Feldenergiedichte in dem Außenbereich, d.h. unterhalb der Segmente geringerer integraler Stromdichte, wird einerseits die Stromdichte im Gewebe unterhalb dieser Bereiche gesenkt (Rückführung des Erregungsstroms) und andererseits die Breite dieses rückführenden Bereiches vergrößert, wodurch sich eine Reduktion des Spannungsabfalls im Gewebe und dadurch eine Vergroßerung des Stimulationseffektes bei gegebener Leistungsaufnahme einstellt. So kann die zur Stimulation nötige Feldenergie und damit auch der Energieverlust reduziert werden.

Die Winkelsegmente mit sich voneinander unterscheidender, integraler Stromdichte können beispielsweise durch eine dezentrale Wicklung des Spulenbereichs verwirklicht werden, bei der aufeinanderfolgende Windungen innerhalb des Winkelsegmentes höherer integraler Stromdichte einen geringeren radialen Abstand voneinander aufweisen als innerhalb des Winkelsegmentes geringerer integraler Stromdichte. Alternativ oder zusätzlich ist es auch möglich, daß die Windungen innerhalb des Winkelsegmentes höherer integraler Stromdichte mit einem geringeren Leiterquerschnitt versehen werden als innerhalb des Winkelsegmentes geringerer integraler Stromdichte.

Die vorstehend erläuterten Vorteile einer Spulenanordnung mit Spulenbereichen unterschiedlicher integraler Stromdichte können mit den davor erläuterten Vorteilen einer Doppelspule kombiniert werden, wenn die Spulenanordnung mindestens zwei Spulenbereiche umfaßt, von denen jeder mindestens zwei Winkelsegmente mit sich voneinander integraler Stromdichte aufweist, wobei die Winkelsegmente höherer integraler Stromdichte radial versetzt nebeneinander angeordnet sind und vorzugsweise tangential ineinander übergehen.

Eine weitere Optimierung des Wirkungsgrades und eine damit einhergehende Reduzierung der Energieverluste kann erzielt werden, wenn die Form der Spule an diejenige des zu stimulierenden Gewebes angepaßt wird, weil dadurch eine verbesserte Kopplung des Spulenfeldes an das Gewebe erreichbar ist. In diesem Zusammenhang hat es sich als besonders zweckmäßig erwiesen, wenn mindestens einer der aus einer Mehrzahl von spiralenartig gewickelten Windungen bestehenden Spulenbereich gewölbt oder in eine gewölbte, vorzugsweise etwa kreiszylindermantelförmige, Form überführbar ist.

Die Magnetspulenanordnung der erfindungsgemäßen Vorrichtung kann ferner durch einen speziellen Aufbau des Spulenleiters verbessert werden, mit dem eine zusätzliche Reduzierung der Spulenverluste unabhängig von der Spulengeometrie erreichbar ist. Der optimale Leiterquerschnitt richtet sich dabei zum einen nach den gewünschten Außenabmessungen der Spule, zum anderen nach einer optimalen Reduzierung der Wirbelstromverluste in der Spule. Im folgenden werden zunächst die theoretischen Grundlagen beider Optimierungskriterien betrachtet. Dabei werden folgende Vereinfachungen vorgenommen:

Die Spulenhöhe wird konstant gehalten. Aus Gründen der besseren Vergleichbarkeit werden auch die Spulenaußenabmessungen konstant gelassen, weil die Gesamtflache etwa mit der Einwirkflache korreliert ist, welche allerdings wesentlich kleiner als die gesamte Spulenfläche ist. Auf diese Weise wird auch die Spulenfläche konstant gehalten. Bei gegebenem Leiterfüllfaktor (Verhältnis von wirksamen Leitervolumen zu Spulenvolumen) ist damit auch das Leitervolumen konstant.

Bei konstanter Gesamtfeldenergie bleiben unter diesen Bedingungen zunächst die Spulenverluste unabhängig von der Windungszahl und damit von der Spuleninduktivität.

Im Hinblick auf die Gesamtverluste einer Stimulationseinrichtung muß die Spuleninduktivität innerhalb der technisch möglichen Grenzen möglichst groß gewählt werden. Durch Vergrößerung der Induktivität reduziert sich nämlich bei konstant gehaltener Stimulationsenergie der Spulenstrom, so daß damit die Zuleitungsverluste sinken. Allerdings muß hierbei die Entladespannung gesteigert werden, welche den technischen Aufwand von Spule und Erregereinrichtung entscheidend mitbestimmt. Neben der Erhöhung der Windungszahl einer einlagigen Spule bietet sich zur Erhöhung der Induktivität auch die Möglichkeit an, die Spule mehrlagig auszuführen. Hierzu kann die Spule aus einzelnen relativ dünnen geschnittenen und voneinander isolierten Blechen aus Leitermaterial hergestellt werden.

Da die optimale Impulsdauer im $\mu$s-Bereich liegt, darf zur Verlustberechnung innerhalb der Stimulationsspule nicht allein ihr Gleichstromwiderstand herangezogen werden. Vielmehr müssen zusätzlich die in den Leitern vom Spulenfeld induzierten Wirbelströme berücksichtigt werden, welche einen beträchtlichen Anteil an den gesamten Spulenverlusten ausmachen. Bei kommerziellen Spulen liegen diese Verluste bei mehr als 50% der Spulenverluste. Durch Feldrechnungen ergibt sich der folgende Zusammenhang für die durch die Wirbelströme in einem Volumen ($V_{paket}$) verursachte Verlustleistung:

$$P_{V.\,Wirbel} = \frac{V_{Paket}}{\chi \cdot d} \cdot \frac{2\alpha \hat{B}^2}{\mu_0^2\, \mu_r^2} \cdot \frac{\sinh(\alpha d)\text{-}\sin(\alpha d)}{\cosh(\alpha d)\text{+}\cos(\alpha d)} \tag{1}$$

mit:

$$\alpha = \sqrt{\frac{1}{2}\, \mu_0 \mu_r \cdot \omega \cdot \chi}$$

und:

$\omega$ = Kieisfrequenz, mit der sich das Feld sinusförmig ändert
$\chi$ = (Leitfähigkeit des Leitermaterials; z.B. Kupfer)
d = Leiterstärke (Filamentstärke)
$\mu_r$ = 1 (relative Permeabilität des Leitermaterials)
$\hat{B}$ = magnetische Flußdichte

Aus diesem Zusammenhang läßt sich folgern, daß sich die Wirbelstromverluste entweder durch Verwendung sehr dünner Leiter oder durch Filamentierung, die im folgenden noch näher erläutert wird, reduzieren lassen. Da durch die Verwendung sehr dünner Leiter die Induktivität stark ansteigt, wird bei der notwendigen Feldenergie eine extrem hohe Kondensatorspannung nötig. Diese sollte aus Gründen des technischen Aufwandes einen Wert von etwa 3kV nicht überschreiten. Daher ist die zweite Möglichkeit, d.h. die Filamentierung zweckmäßiger. Unter Filamentierung versteht man das Einbringen dünner, isolierender Trennschnitte oder das Aufspalten des Leiters in dünne voneinander isolierte, elektrisch parallel geschaltete Teilleiter.

Da allerdings die Filamentierungsschnitte aus technischen Gründen immer eine endliche Stärke aufweisen und somit bei Filamentierung mit geringem Filamentabstand den Füllfaktor reduzieren, läßt sich bei gegebener Frequenz, Feldstärke und Spulengeometrie immer eine optimale Filamentierungsfeinheit im Hinblick auf minimale Verlustenergie finden.

Die gesamten Spulen-Stromwärmeverluste setzen sich zusammen aus dem ohmschen Kupferverlusten (unter Verwendung des Gleichstromwiderstands der Spule) und den Wirbelstromverlusten:

$$P_{V.\,gesamt} = P_{V.\,Wirbel} + P_{V.\,Cu} = P_{V.\,Wirbel} + \frac{1}{2} \cdot \frac{C}{L}\hat{U}^2 \cdot R_{i.DC} \tag{2}$$

mit: $\hat{U}$ = Entladespannung
und C = Kapazität des Schwingkreiskondensators
Bei Verwendung von konventionellem Leitermaterial entstehen bei dezentraler Windungsanordnung im Außenbereich der Spule große Zwischenräume.

Steht allerdings nicht das Gewicht der Spule sondern nur ihr Volumen bei der Anwendung im Vordergrund, so kann eine optimale Füllung mit Leitermaterial hier durch eine über den Winkel veränderliche Leiterbreite erreicht werden. Durch diese Maßnahme kann der Innenwiderstand und damit auch der Stromwärmeverlust der Spulen nochmals beträchtlich reduziert werden. Dabei muß allerdings auch die Zahl der Filamente über den Winkel geändert werden, wobei der Leiter innerhalb eines Winkelsegmentes geringerer integraler Stromdichte vorzugsweise mehr Teilleiter aufweist als innerhalb eines Winkelsegmentes höherer integraler Stromdichte.

Insbesondere bei Einsatz von erfindungsgemäßen Vorrichtungen mit filamentierten, d.h. eine Mehrzahl von elektrisch voneinander isolierten und parallel geschalteten Teilleitern aufweisenden Leitern, können die Spulenverluste weiter reduziert werden, wenn mindestens zwei, vorzugsweise jedoch sämtliche parallel geschalteten Teilleiter während der Erzeugung der Stromimpulse etwa von dem gleichen magnetischen Fluß durchsetzt werden. Diese Verbesserung beruht auf der Erkenntnis, daß in parallel geschalteten Teilleitern, die unterschiedliche magnetische Flüsse einschließen, bei einer Stromänderung und damit einhergehenden Änderung der magnetischen Flüsse auch unterschiedliche Spannungen induziert werden. Da durch die Parallelschaltung der einzelnen Teilleiter gleiche Spannungsabfälle über allen Teilleitern erzwungen werden, müssen sich Ausgleichsströme bilden. Detaillierte Rechnungen und Messungen haben gezeigt, daß diese Ausgleichsströme erhebliche Zusatzverluste erzeugen.

Diese Betrachtung gilt für massive Leiter entsprechend. Hier erzeugen die Ausgleichsströme ungleichmäßige Stromdichteverteilungen, so daß der effektive Wirkungsquerschnitt des stromführenden Leiters stark verringert wird. Durch die beschriebene Weiterbildung der erfindungsgemäßen Vorrichtung, bei der die einzelnen Teilleiter so angeordnet werden, daß sie von etwa gleichen magnetischen Flüssen durchsetzt werden, können die beschriebenen Zusatzverluste zumindest teilweise vermieden werden.

Insbesondere bei einlagigen` eine Mehrzahl von nebeneinander angordneten Teilleitern aufweisenden Spulen kann eine Anordnung der parallel geschalteten Teilleiter, bei der mindestens zwei der parallel geschalteten Teilleiter während der Erzeugung der Stromimpulse etwa von dem gleichen magnetischen Fluß durchsetzt werden, besonders einfach verwirklicht werden, in dem die einzelnen isolierten Teilleiter verdrillt bzw. verröbelt werden.

Die beschriebenen Zusatzverluste treten auch bei Magnetspulenanordnungen mit einer Mehrzahl von bezüglich einer gemeinsamen Spulenachse axial versetzt übereinander angeordneten Spulenbereichen auf, weil das Magnetfeld mit zunehmendem Abstand von dem mittleren Spulenbereich immer kleiner wird. Auch bei dieser Anordnung können die Spulenverluste verringert werden, in dem die Leiter der einzelnen übereinander angeordneten Spulenbereiche miteinander verdrillt werden. Aus Platz- und Konstruktionsgründen hat es sich jedoch als besonders zweckmäßig erwiesen, wenn für gleiche flußwirksame Flächen in den einzelnen Spulenbereichen gesorgt wird. Das kann dadurch erreicht werden, daß die einzelnen axial versetzt übereinander angeordneten Teilleiter in entsprechenden Windungsbereichen unterschiedliche Krümmungsradien aufweisen. Alternativ oder zusätzlich können die Zusatzverluste in Magnetspulenanordnungen mit einer Mehrzahl von übereinander angeordneten Spulenbereichen dadurch vermindert werden, daß mindestens zwei dieser Spulenbereiche sich voneinander unterscheidende Windungszahlungen aufweisen.

Bei Magnetspulenanordnungen mit zwei nebeneinander angeordneten, gleichsinnig gewickelten Spulenbereichen können die Zusatzverluste verringert werden, wenn die Teilleiter der äußeren Windungen der einzelnen Spulenbereiche tangential ineinander übergehen. Dann sind die von den einzelnen Leitern eingeschlossenen Flüsse aus Symmetriegründen etwa gleich groß.

Die beschriebenen Maßnahmen zur Vermeidung der erläuterten Zusatzverluste können auch miteinander kombiniert werden, z.B. in Form von mehrlagigen Magnetspulenanordnungen aus miteinander verdrillten Teilleitern mit Zusatzwindungen in den äußeren Spulenbereichen.

Bei vielen Anwendungen, bei denen die Magnetspulenanordnung durch repetitive Stimulation mit Impulsen großer Stärke beaufschlagt wird, kann das Gewicht bzw. die Große der Magnetspulenanordnung dadurch besonders kleingehalten werden, daß sie nur für den Kurzzeitbetrieb konzipiert wird und dafür ihre Wärmekapazität durch spezielle Maßnahmen vergrößert wird. Wird die Magnetspulenanordnung innerhalb einer relativ kurzen Zeit mit einer bestimmten Anzahl von Stromimpulsen belastet, erwärmt sie sich entsprechend ihrer Wärmekapazität und kann dann über einen längeren Zeitraum wieder abgekühlt werden. Der Arbeitspunkt von Magnetspulenanordnungen üblicher Vorrichtungen zur Stimulation von Körpergewebe ist durch die Verwendung am menschlichen Körper allerdings stark eingeschränkt. Er liegt etwa zwischen Zimmertemperatur und Körpertemperatur.

Die Magnetspulenanordnung der erfindungsgemäßen Vorrichtung kann innerhalb dieses Temperaturbereiches eine besonders große Warmemenge aufnehmen, wenn sie einen innerhalb des Temperaturbereichs zwischen 0 und 50°C, vorzugsweise innerhalb des Temperaturbereichs zwischen 18° und 40°C, besonders bevorzugt innerhalb des Temperaturbereichs zwischen 20 und 37°C, einen Phasenübergang durchlaufenden Latentwärmespeicher aufweist. Solche Phasenübergänge können z.B. Schmelz-, Kristallisations- oder Losungsvorgange, aber auch Modifikationsumwandlungsprozesse sein.

Nachstehend wird die Erfindung unter Bezugnahme auf die Zeichnung, auf die hinsichtlich aller erfindungswesentlichen und in der Beschreibung nicht naher herausgestellten Einzelheiten ausdrücklich verwiesen wird, erläutert. In der Zeichnung zeigt:

Fig. 1    eine Schaltungsanordnung einer erfindungsgemäßen Vorrichtung,

Fig. 2    eine Magnetspulenanordnung gemäß einer ersten Ausführungsform der Erfindung,

Fig. 3    eine Magnetspulenanordnung gemäß einer zweiten Ausführungsform der Erfindung,

Fig. 4    eine Magnetspulenanordnung gemaß einer dritten Ausführungsform der Erfindung,

Fig. 5    eine Magnetspulenanordnung gemäß einer vierten Ausführungsform der Erfindung,

Fig. 6    eine Magnetspulenanordnung gemäß einer fünften Ausführungsform der Erfindung,

Fig. 7    eine Magnetspulenanordnung gemaß einer sechsten Ausführungsform der Erfindung und

Fig. 8    eine Magnetspulenanordnung gemäß einer siebten Ausführungsform der Erfindung.

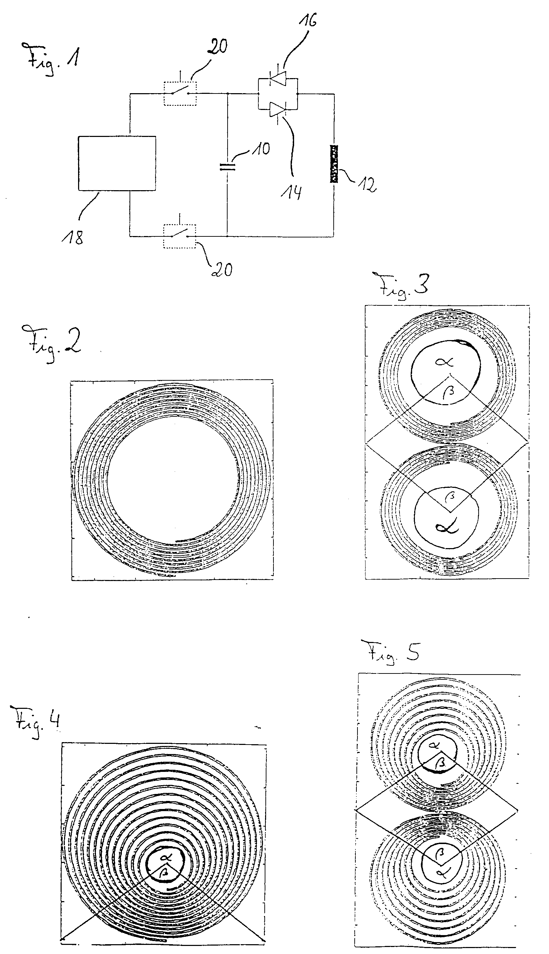

Die in Fig. 1 dargestellte Schaltungsanordnung besteht im wesentlichen aus einem einen Kondensator 10 und eine Spulenanordnung 12 aufweisenden Schwingkreis. Im Entladekreis des Kondensators 10 sind zwei gegensinnig parallel zueinander angeordnete Thyristoren 14 und 16 vorgesehen. Durch entsprechende Ansteuerung dieser Thyristoren 14 und 16 mit einer in der Zeichnung nicht dargestellten Steuereinrichtung können in der Magnetspulenanordnung 12 Stromimpulse in Form von Sinushalbwellen oder Sinusvollwellen erzeugt werden.

Zur Erzeugung einer Sinushalbwelle wird die Entladung des Kondensators 10 über die Magnetspulenanordnung 12 durch entsprechende Ansteuerung eines der Thyristoren freigegeben. Der Entladung des Kondensators 10 über die Magnetspulenanordnung 12 folgt ein erneutes Aufladen des Kondensators 10 nach Art eines gewohnlichen Schwingkreises. Eine weitere Entladung des Kondensators 10 kann jedoch durch entsprechende Ansteuerung der Thyristoren 12 und 14 verhindert bzw. verzögert werden.

Wahrend der Entladung des Kondensators 10 auftretende Verluste können mit einer über Schalter 20 an den Kondensator 10 anschließbaren Ladeschaltung 18 ausgeglichen werden. Aus unterschiedlichen medizinischen Untersuchungen hat sich ergeben, daß die Ansteuerung der Magnetspulenanordnung mit einem Stromimpuls in Form einer Sinushalbwelle oder Sinusvollwelle zum Erhalt der gewünschten Stimulation von menschlichen Nerven- oder Muskelgewebe besonders geeignet ist. Ein weiterer wesentlicher Vorteil dieser Impulsform ist darin zu sehen, daß die Feldenergie der Spule nahezu vollständig durch Rückladung in den Kondensator wieder zurückgewonnen werden kann.

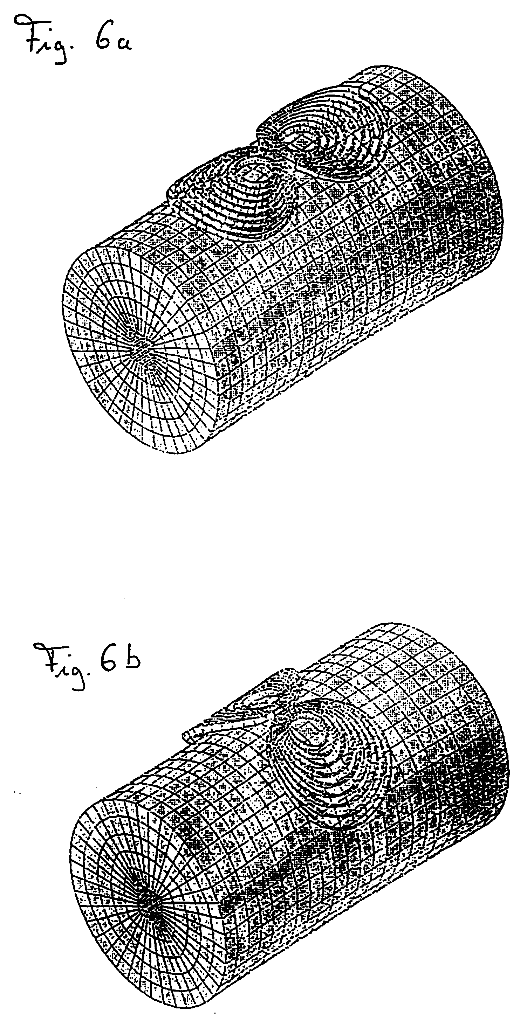

Die in Fig. 2 dargestellte Magnetspulenanordnung besteht aus einer einlagig, als Spirale gewickelten, flachen Luftspule. Diese Spulenanordnung ist einerseits besonders leicht und andererseits so geformt, daß alle stromdurchflossenen Windungen in unmittelbare Nahe zum zu stimulierenden Gewebe gebracht werden können.

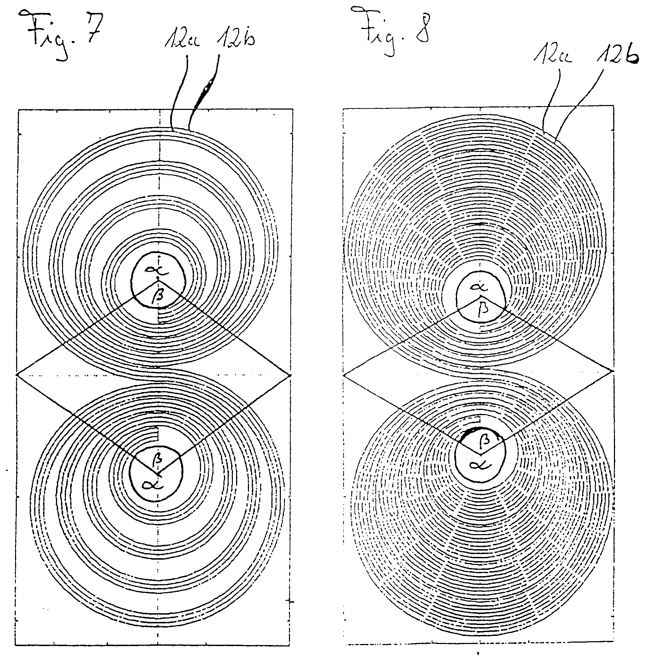

Die in Fig. 3 dargestellte Magnetspulenanordnung besteht aus einer Doppelspule mit zwei gleichsinnig als Spirale gewickelten, flachen Luftspulen, die bezüglich einer Spulenachse radial versetzt nebeneinander angeordnet sind und deren äußere Windungen tangential ineinander übergehen. Mit der in Fig. 3 dargestellten Doppelspule wird unterhalb der unmittelbar nebeneinander angeordneten Winkelsegmente β der einzelnen als Spirale gewickelten Spulenbereiche das größte Feld und damit die stärkste Stimulationswirkung erzeugt, da sich hier die größte Anzahl gleichsinnig durchflossener Leiter direkt nebeneinander befinden. Da die Stromverteilung des in einem unterhalb der Magnetspulenanordnung gemäß Fig. 3 angeordneten Körpergewebe induzierten Stromes etwa die gleiche Form wie die Leiteranordnung in der Magnetspulenanordnung aufweist, findet unterhalb der äußeren Winkelsegmente α eine Rückführung des induzierten Gewebestromes mit gegenüber dem unterhalb der inneren Winkelsegmente β induzierten Gewebestrom reduzierter Stromdichte statt.

Die in Fig. 4 dargestellte Magnetspulenanordnung besteht ähnlich wie die Magnetspulenanordnung nach Fig. 2 aus einer einlagigen, als Spirale gewickelten flachen Luftspule. Die Magnetspulenanordnung nach Fig. 4 weist jedoch eine dezentrale Anordnung der einzelnen Leiterwindungen auf, so daß aufeinanderfolgende Windungen innerhalb eines Winkelsegmentes β einen deutlich geringeren radialen Abstand voneinander aufweisen als innerhalb eines Winkelsegmentes α. Dadurch wird innerhalb des Winkelsegmentes β eine höhere integrale Stromdichte erzeugt als innerhalb des Winkelsegmentes α. Das hat eine Erhöhung der innerhalb des unter dem Winkelsegment β angeordneten Körpergewebe erzeugten Stromdichte und eine Verringerung der innerhalb des unterhalb des Winkelsegmentes α angeordneten Körpergewebes induzierten rückfuhrenden Stromdichte zur Folge. Auf diese Weise wird die Breite des rückführenden Bereiches innerhalb des Körpergewebes vergrößert, wodurch sich eine Reduktion des Spannungsabfalls im Gewebe und dadurch eine Vergrößerung des Stimulationseffektes einstellt.

Die Magnetspulenanordnung nach Fig. 5 stellt eine Kombination aus der Magnetspulenanordnung nach Fig. 4 und der Magnetspulenanordnung nach Fig. 3 dar. Mit dieser dezentralen Doppelspule kann im Vergleich zur zentralen Doppelspule eine bessere Fokalität der Stimulation erreicht werden. Die Absenkung der Stromdichte in den Außenbereichen $\alpha$ dieser dezentralen Doppelspule reduziert weiter das Auftreten von sogenannten Nebenmaxima, d.h. Orten an den Spulenrändern, an denen ebenfalls eine Stimulationswirkung auftritt oder auftreten kann.

Die in Fig. 6 dargestellte Magnetspulenanordnung entspricht im wesentlichen der anhand der Fig. 5 erläuterten dezentralen Doppelspule. Die Magnetspulenanordnung nach Fig. 6 ist jedoch zur besseren Anpassung ihrer Form an das zu stimulierende Gewebe gewölbt, bzw. in eine gewölbte Form überführt. Dabei ist die in Fig. 6a dargestellte Doppelspule mit dezentraler Leiteranordnung in eine Kreiszylindermantelform überführt, bei der die einzelnen Spulenbereiche längs der Kreiszylinderachse nebeneinander angeordnet sind, während die einzelnen Spulenbereiche bei der in Fig. 6b dargestellten Magnetspulenanordnung in Umfangsrichtung einer Kreiszylindermantelfläche nebeneinander angeordnet sind.

Anhand der Fig. 7 und 8 wird nachstehend eine im Hinblick auf die Reduzierung der Spulenverluste optimierter Aufbau der zur Herstellung der Magnetspulenanordnung verwendeten Leiter erläutert. Wie in der Beschreibungseinleitung bereits erläutert, können die Wirbelstromverluste einer Magnetspulenanordnung wirksam reduziert werden, wenn diese aus einem eine Mehrzahl von elektrisch voneinander isolierten und parallel geschalteten Teilleitern aufweisenden filamentierten Leiter gebildet ist: Im Hinblick auf den Erhalt eines möglichst geringen Gewichtes der Magnetspulenanordnung hat sich dabei die in Fig. 7 dargestellte Magnetspulenanordnung in Form einer Doppelspule mit dezentralen Windungen als besonders zweckmäßig erwiesen, bei der Leiterfilamente 12b durch isolierende Filamentierungsschnitte 12a voneinander getrennt sind.

Bei Verwendung von konventionellem Leitermaterial entstehen bei dezentraler Windungsanordnung im Außenbereich der Spule große Zwischenräume. Steht nicht das Gewicht der Spule sondern ihr Volumen bei der Anwendung im Vordergrund, so kann eine optimale Füllung mit Leitermaterial hier durch eine über den Winkel veränderliche Leiterbreite der in Fig. 8 dargestellten Art erreicht werden. Durch diese Maßnahme kann der Innenwiderstand und damit auch der Stromwärmeverlust der Spule nochmals beträchtlich reduziert werden. Allerdings muß dabei auch die Zahl der Filamente über den Winkel verändert werden, wie in Fig. 8 dargestellt.

Bei den in den Fig. 7 und 8 dargestellen Ausführungsformen der Erfindung gehen die Leiterfilamente 12b der äußeren Windungen der nebeneinander angeordneten Spulenbereiche tangential ineinander über. Dadurch wird erreicht, daß die von den einzelnen Leiterfilamenten eingeschlossenen Flüsse etwa gleich groß sind.

## Patentansprüche

1. Vorrichtung zur Stimulation von Körpergewebe mit einer auf eine Körperoberfläche aufsetzbaren Magnetspulenanordnung (12) und einer zur Erzeugung von - Stromimpulsen in der Magnetspulenanordnung betreibbaren Erregereinrichtung (10, 14, 16), dadurch gekennzeichnet, daß die Magnetspulenanordnung (12) mindestens einen aus einer Mehrzahl von spiralenartig gewickelten Windungen bestehenden Spulenbereich aufweist.

2. Vorrichtung nach Anspruch 1, dadurch gekennzeichnet, daß die Magnetspulenanordnung mindestens zwei Spulenbereiche aufweist, von denen jeder aus einer Mehrzahl von spiralenartig gewickelten Windungen besteht.

3. Vorrichtung nach Anspruch 2, dadurch gekennzeichnet, daß mindestens zwei Spulenbereiche bezüglich einer gemeinsamen Spulenachse axial versetzt übereinander angeordnet sind.

4. Vorrichtung nach Anspruch 2 oder 3, dadurch gekennzeichnet, daß mindestens zwei Spulenbereiche bezüglich einer Spulenachse radial versetzt nebeneinander angeordnet sind.

5. Vorrichtung nach Anspruch 4, dadurch gekennzeichnet, daß die Erregereinrichtung zur Erzeugung gleichsinniger Stromimpulse in einander unmittelbar benachbarten Segmenten ($\beta$) der nebeneinander angeordneten Spulenbereiche betreibbar ist.

6. Vorrichtung nach Anspruch 4 oder 5, dadurch gekennzeichnet, daß die nebeneinander angeordneten Spulenbereiche ($\beta$) gleichsinnig gewickelt sind und die äußeren Windungen dieser Spulenbereich vorzugsweise tangential ineinander übergehen.

7. Vorrichtung nach einem der vorhergehenden Ansprüche, dadurch gekennzeichnet, daß mindestens einer der aus spiralenartig gewickelten Windungen bestehenden Spulenbereiche mindestens zwei Winkelsegmente ($\alpha$, $\beta$) mit sich voneinander unterscheidender integraler Stromdichte aufweist.

8. Vorrichtung nach Anspruch 7, dadurch gekennzeichnet, daß die Windungen des zwei Segmente mit sich voneinander unterscheidender integraler Stromdichte aufweisenden Spulenbereichs derart dezentral gewickelt sind, daß aufeinanderfolgende Windungen innerhalb des Wickelsegmentes ($\beta$) höherer integraler Stromdichte einen geringeren radialen Abstand voneinander aufweisen als innerhalb des Wickelsegmentes ($\alpha$) geringerer integraler Stromdichte.

9. Vorrichtung nach Anspruch 7 oder 8, dadurch gekennzeichnet, daß die Windungen innerhalb des Wickelsegmentes ($\beta$) höherer integraler Stromdichte einen geringeren Leiterquerschnitt aufweisen als innerhalb des Wickelsegmentes ($\alpha$) geringerer integraler Stromdichte.

10. Vorrichtung nach einem der Ansprüche 5 bis 9, dadurch gekennzeichnet, daß die Magnetspulenanordnung mindestens zwei Spulenbereiche umfaßt, von denen jeder mindestens zwei Winkelsegmente ($\alpha$, $\beta$) mit sich voneinander unterscheidender integraler Stromdichte aufweist, wobei die Winkelsegmente ($\beta$) höherer integraler Stromdichte radial versetzt nebeneinander angeordnet sind, vorzugsweise tangential ineinander übergehen.

11. Vorrichtung nach einem der vorhergehende Ansprüche, dadurch gekennzeichnet, daß mindestens einer der aus einer Mehrzahl von spiralenartig gewickelten Windungen bestehenden Spulenbereiche gewölbt oder in eine gewölbte, vorzugsweise etwa kreiszylindermangelförmige, Form überfuhrbar ist.

12. Vorrichtung nach einem der vorhergehenden Ansprüche, dadurch gekennzeichnet, daß mindestens einer der Spulenbereiche aus einem eine Mehrzahl von elektrisch voneinander isolierten und Parallel geschalteten Teilleitern (12a) aufweisenden filamentierten Leiter gebildet ist.

13. Vorrichtung nach Anspruch 12, dadurch gekennzeichnet, daß mindestens zwei, vorzugsweise alle parallel geschalteten Teilleiter während der Erzeugung der Stromimpulse etwa von dem gleichen magnetischen Fluß durchsetzt werden.

14. Vorrichtung nach Anspruch 13, dadurch gekennzeichnet, daß mindestens zwei Teilleiter miteinander verdrillt bzw. verröbelt sind.

15. Vorrichtung nach Anspruch 13 oder 14, dadurch gekennzeichnet, daß mindestens zwei Teilleiter in Richtung einer gemeinsamen Spulenachse axial versetzt übereinander angeordnet sind und in einander entsprechenden Windungsbereichen unterschiedliche Krümmungsradien aufweisen.

16. Vorrichtung nach Anspruch 6 und einem der Ansprüche 12 bis 15, dadurch gekennzeichnet, daß die Teilleiter der äußeren Windungen tangential ineinander übergehen.

17. Vorrichtung nach Anspruch 3, dadurch gekennzeichnet, daß mindestens zwei der übereinander angeordneten Spulenbereiche sich voneinander unterscheidende Windungszahlen aufweisen.

18. Vorrichtung nach Anspruch 9 und einem der Ansprüche 12 bis 17, dadurch gekennzeichnet, daß der Leiter innerhalb des Winkelsegmentes ($\alpha$) geringerer integraler Stromdichte mehr parallel geschaltete Teilleiter (12b) aufweist als innerhalb des Winkelsegmentes ($\beta$) höherer integraler Stromdichte.

19. Vorrichtung nach einem der vorhergehenden Ansprüche, dadurch gekennzeichnet, daß die Magnetspulenanordnung (12) einen innerhalb des Temperaturbereichs zwischen 0 und 50°C, vorzugsweise innerhalb des Temperaturbereichs zwischen 18 und 40°C, besonders bevorzugt innerhalb des Temperaturbereichs zwischen 20 und 37°C, einen Phasenübergang durchlaufenden Latentwärmespeicher aufweist.

20. Vorrichtung nach einem der vorhergehenden Ansprüche, dadurch gekennzeichnet, daß die Erregereinrichtung einen über die Magnetspulenaordnung (12) entladbaren Kondensator (120) aufweist.

21. Vorrichtung nach Anspruch 20, dadurch gekennzeichnet, daß die Erregereinrichtung mindestens einen zum Freigeben der Entladung des Kondensators (10) über die Magnetspulenanordnung (12) ansteuerbaren Thyristor (14, 16) aufweist.

22. Vorrichtung nach Anspruch 21, dadurch gekennzeichnet, daß die Erregereinrichtung mindestens zwei parallel und gegensinnig geschaltete Thyristoren (14, 16) aufweist, die unabhängig voneinander zum Freigeben der Entladung des Kondensators (10) über die Magnetspulenanordnung (12) ansteuerbar sind.

Fig. 1

Fig. 2

Fig. 3

Fig. 4

Fig. 5

Fig. 6a

Fig. 6b

Fig. 7

Fig. 8